# FASCICULE DE BREVET EUROPEEN

(11) **EP 3 890 805 B1**
(45) Date de publication et mention de la délivrance du brevet: **26.03.2025**
(21) Numéro de dépôt: 20705239.0
(22) Date de dépôt: 23.01.2020
(51) Int. Cl.: A61M 5/20

(54) **DISPOSITIF D'INJECTION MANUELLE ASSISTÉE POUR INJECTER UNE COMPOSITION CONTENUE DANS UN RÉCIPIENT**
VORRICHTUNG ZUR UNTERSTÜTZTEN MANUELLEN INJEKTION ZUR INJEKTION EINER IN EINEM BEHÄLTER AUFBEWAHRTEN ZUSAMMENSETZUNG
ASSISTED MANUAL INJECTION DEVICE FOR INJECTING A COMPOSITION CONTAINED IN A CONTAINER

(30) Priorité: 24.01.2019 FR 1900613
(43) Date de publication de la demande: 13.10.2021
(73) Titulaire: Eveon, 38330 Montbonnot-Saint-Martin (FR)
(72) Inventeur: WATTELLIER, Arnaud, 38190 Laval (FR); BEARD, Jean-Claude, 01300 Belley (FR); DEHAN, Christophe, 38330 Saint-Ismier (FR)
(74) Mandataire: Icosa
(86) Numéro de dépôt international: PCT/FR2020/050093
(87) Numéro de publication internationale: WO 2020/152424

(56) Documents cités:
- EP-A1- 2 918 299
- WO-A1-2011/113806
- WO-A1-2017/201185
- US-A- 6 159 161
- US-A1- 2005 004 514
- US-A1- 2017 312 430

## Description

### DOMAINE DE L'INVENTION

La présente invention concerne un dispositif d'injection manuelle assistée pour injecter une composition contenue dans un récipient.

### ETAT DE LA TECHNIQUE

Les dispositifs d'injection préremplis sont des récipients couramment utilisés pour l'injection de compositions pharmaceutiques ou cosmétiques, et comprennent notamment des seringues, des cartouches, et des auto-injecteurs. Ces récipients comprennent typiquement un piston d'étanchéité apte à se déplacer en translation dans le récipient, le récipient étant rempli avec une composition pharmaceutique ou cosmétique afin de fournir aux opérateurs de santé des dispositifs d'injection prêts à l'emploi pour traiter les patients.

Un récipient présente une forme cylindrique, et comprend une extrémité proximale ouverte par laquelle peut être inséré le piston afin de refermer de manière étanche un volume interne du récipient renfermant la composition à injecter, une extrémité distale usuellement munie d'une aiguille par laquelle la composition est injectée, et une surface latérale qui s'étend entre l'extrémité proximale et l'extrémité distale du récipient. En pratique, le piston est apte à se déplacer suite à un effort mécanique exercé sur celui-ci par une tige de piston mobile en translation dans le récipient, depuis l'extrémité proximale du récipient vers son extrémité distale, afin d'injecter la composition. De tels récipients préremplis standards dans l'industrie médicale sont par exemples les seringues préremplies (« pre-filled syringes », en anglais, acronyme PFS) avec ou sans aiguille attachée à son extrémité distale, ainsi que les cartouches. Les cartouches présentent en outre un septum qui sera percé par une aiguille à leur extrémité distale pour l'injection.

Par rapport aux dispositifs d'injection vides qui sont remplis juste avant l'injection avec des compositions préalablement stockées dans des flacons (« vials » en anglais), l'utilisation de dispositifs d'injection préremplis présente plusieurs avantages. En particulier, en limitant les étapes de préparation avant l'injection, les dispositifs d'injection préremplis sont plus simples à utiliser, permettent d'améliorer l'asepsie, et contribuent à réduire les erreurs de dosage. Les dispositifs d'injection préremplis encouragent et simplifient l'administration ou l'auto-administration de la composition, ce qui réduit le coût des soins et facilite le traitement. Enfin, les dispositifs d'injection préremplis réduisent les pertes de composition pharmaceutique ou cosmétique qui peuvent survenir lorsque la composition est transférée depuis un flacon de stockage dans un dispositif d'injection de type non-préremplis. Il en résulte une réduction des coûts d'achat et de chaîne d'approvisionnement.

Dans certains cas, l'injection de la composition contenue dans le récipient avec un dispositif d'injection manuelle, tel qu'une seringue, peut être difficile à réaliser, à cause de l'effort qui doit être exercé sur la tige de piston pour expulser la composition du récipient. Cela se produit, par exemple, lorsque sont associés une aiguille de petit diamètre, un volume déterminé à injecter dans un temps impartit et certaines caractéristiques rhéologiques de la composition telle qu'une viscosité élevée, et/ou lorsque l'injection est réalisée manuellement par un utilisateur qui est incapable d'appuyer suffisamment fort sur la tige de piston avec ses doigts.

Ainsi, pour l'injection manuelle de gels, ciments, suspensions et autres produits très visqueux, l'opérateur doit exercer une grande force sur le piston de la seringue d'injection pour assurer le passage du matériau dans une aiguille. Il en résulte :
- une vitesse d'injection qui diminue quand on réduit le diamètre de l'aiguille, par exemple lors de l'injection de gels de comblements de ride ou de visco-supplémentation articulaire,
- une fatigue musculaire importante, voire une limitation du nombre d'actes qui peuvent être réalisés successivement par un même opérateur,
- une réduction de la précision ou un tremblement dans la réalisation des actes annexes que doit exercer l'opérateur alors qu'il procède à l'injection, qui peuvent influencer le placement, la profondeur, ou le déplacement de l'aiguille dans les tissus d'injection.

Pour les dispositifs d'injection manuelle, des fabricants ont ajouté des surfaces ou des pièces de préhension aux dispositifs d'injection, tels que des brides de grande taille contre lesquelles l'index et le majeur ou le pouce peuvent venir en appui lorsque le dispositif est pris en main de manière tridactyle, le pouce faisant pression contre l'extrémité proximale de la tige de piston afin de pousser ladite tige de piston en direction distale pour injecter la composition. Ont également été ajoutés des dispositifs passifs annexes dans lesquels on glisse la seringue.

Les surfaces et les pièces de préhension ne résolvent que la facilité de préhension, c'est-à-dire l'ergonomie, ou la capacité mécanique à exercer une grande force en augmentant la surface de préhension. Les problèmes relatifs à la force maximale que peut exercer la main, la fatigue musculaire ou les tremblements musculaires qui résultent de la contraction musculaire de la main ne sont cependant pas résolus.

Des dispositifs à multiplication d'effort ont également été développés.

Cependant, dans ces dispositifs à multiplication d'effort, l'effort que doit appliquer l'utilisateur pour réaliser l'injection est réduit, mais la variation de contraction musculaire d'injection avec la reprise de course (relâcher le moyen d'injection, généralement une gâchette, qui revient à son origine, puis reprendre l'appui) nécessite une coordination délicate lors d'une injection avec déplacement progressif de l'aiguille, par exemple l'injection d'un gel de comblement de ride ou d'un ciment osseux, et induit un déplacement parasite de la main qui peut aboutir à une possible blessure du patient par l'aiguille.

Les documents US 6 159 161 A, WO 2011/113806 A1 et WO 2017/201185 A1 décrivent des dispositifs d'injection.

### EXPOSE DE L'INVENTION

L'invention a donc pour but de remédier aux inconvénients de l'art antérieur.

A cette fin, la présente invention concerne un dispositif d'injection manuelle assistée pour injecter une composition contenue dans un récipient, dans lequel le récipient est muni à son extrémité distale d'une aiguille et contient un piston, le dispositif d'injection comprenant :
- un corps comprenant au moins une zone de préhension par laquelle le corps peut être pris en main par un utilisateur,
- un élément de maintien du récipient configuré pour recevoir et maintenir le récipient de manière fixe par rapport au corps,
- un système électromécanique comprenant :
   - une tige de piston apte à se déplacer en translation dans le récipient par rapport au corps du dispositif afin de déplacer le piston,
   - un système d'actionnement de la tige de piston,
   - un processeur apte à communiquer avec le système d'actionnement,

le dispositif étant principalement caractérisé en ce qu'il comprend au moins un capteur de force positionné sur le corps au niveau d'une zone de préhension, le capteur de force étant configuré pour recevoir en entrée un effort mécanique exercé sur ledit capteur par l'utilisateur lors de l'injection de la composition, et pour transmettre au processeur un signal de sortie qui est fonction dudit effort mécanique,
et le processeur est configuré pour envoyer une instruction au système d'actionnement en fonction du signal de sortie du capteur pour actionner la tige de piston selon une loi de commande prédéterminée, afin d'ajuster l'effort mécanique devant être exercé par l'utilisateur sur la tige de piston pour injecter la composition.

Selon d'autres aspects, le dispositif d'injection présente les différentes caractéristiques suivantes prises seules ou selon leurs combinaisons techniquement possibles :
- le système d'actionnement comprend un système de multiplication d'effort configuré pour fournir à la tige de piston un effort mécanique F2 qui est un multiple de l'effort F1 exercé par l'utilisateur sur le capteur, et dans lequel la loi de commande est une loi de multiplication d'effort ;
- la loi de commande définit un déplacement de la tige de piston dans une direction proximale selon une distance D1 prédéterminée lorsque l'injection est interrompue et que l'effort mécanique F1 exercé par l'utilisateur sur le capteur est inférieur à une valeur seuil F1ad déterminée pendant une durée déterminée Δt1 ;
- la tige de piston présente une position de démarrage, et la loi de commande définit un déplacement de la tige de de piston dans une direction proximale selon une distance D2 prédéterminée lorsque l'injection est terminée et que l'effort mécanique F1 exercé par l'utilisateur sur le capteur est inférieur à une valeur seuil F1ad déterminée pendant une durée déterminée Δt2 ou lorsque la tige de piston est en fin de course distale ;
- l'aiguille étant positionnée dans une zone sous-cutanée du corps humain, la loi de commande définit un déplacement de la tige de piston dans une direction proximale selon une distance D3 prédéterminée afin de réaliser une aspiration calibrée d'un volume déterminé d'un fluide humain, afin de détecter la présence éventuelle de sang dans l'aiguille ;
- le dispositif d'injection comprend en outre un moyen d'activation sélectif permettant à l'utilisateur de sélectivement activer ou désactiver le pilotage de la tige de piston par le processeur via la loi de commande ;
- le moyen d'activation sélectif comprend un moyen de réglage permettant à l'utilisateur de choisir un seuil F1s de déclenchement, de sorte que le pilotage de la tige de piston par le processeur via la loi de commande se déclenche seulement lorsque le l'effort exercé par l'utilisateur sur le capteur est supérieur ou égal au seuil F1s de déclenchement ;
- le corps du dispositif d'injection comprend au moins deux zones de préhension sur une surface distale du corps du dispositif, dont au moins une des zones de préhension est munie d'un capteur et est apte à recevoir l'appui de l'index, du majeur, de l'annulaire, ou de l'auriculaire de l'utilisateur, et la tige de piston comprend une extrémité proximale comprenant une zone de préhension, ladite zone de préhension étant munie d'un capteur et étant apte à recevoir l'appui du pouce de l'utilisateur, pour permettre une prise en main du dispositif de type seringue;
- la zone de préhension de la tige de piston est positionnée sur une gâchette solidaire de la tige de piston et qui s'étend de manière oblique ou perpendiculaire à la tige de piston depuis une position distale par rapport à l'extrémité proximale de ladite tige de piston ;
- le dispositif d'injection comprend au moins trois zones de préhension agencées sur le corps du dispositif autour d'un axe longitudinal du dispositif, dont au moins une des zones de préhension est munie d'un capteur, lesdites zones de préhension étant aptes à recevoir respectivement l'appui de l'index, du majeur, ou du pouce de l'utilisateur pour permettre une prise en main du dispositif de type stylo ;
- l'élément de maintien du récipient comprend un capot pivotant muni d'un orifice apte à recevoir au moins l'extrémité proximale du récipient, le capot pivotant étant apte à pivoter entre une position d'injection dans laquelle l'orifice est aligné avec la course de la tige de piston, et une position libre dans laquelle l'orifice est désaxé par rapport à la course de la tige de piston et accessible par l'utilisateur pour permettre l'insertion du récipient dans ledit orifice ;
- l'élément de maintien du récipient comprend en outre une pluralité de bagues interchangeables munies d'orifices de différents diamètres adaptés pour recevoir des récipients correspondants de différents diamètres ;
- le capot pivotant est réalisé en plusieurs parties, dont au moins une première partie intégrale avec le corps du dispositif, et au moins une deuxième partie mobile apte à pivoter entre la position d'injection et la position libre, les deux parties du capot pivotant formant ainsi une pince à écartement variable permettant d'accueillir et d'enserrer le récipient afin de le maintenir en position fixe ;
- la deuxième partie mobile est apte à être détachée du corps du dispositif ;
- la deuxième partie mobile est codée couleur pour être identifiable par l'utilisateur ou est munie d'un dispositif de reconnaissance et de lecture électronique pour indiquer au processeur les caractéristiques géométriques ou rhéologiques du récipient et de son contenu afin que le dispositif de reconnaissance puisse automatiquement calculer la dose de composition délivrée pour un déplacement unitaire de la tige de piston ;
- le système électromécanique comprend une mémoire dans laquelle sont enregistrées des lois de commande et/ou des valeurs seuils ;
- le dispositif d'injection comprend en outre une interface utilisateur permettant à l'utilisateur d'enregistrer et de sélectionner les paramètres de fonctionnement du dispositif, et d'avoir un retour d'information du dispositif sur lesdits paramètres de fonctionnement lors de l'utilisation du dispositif.

### DESCRIPTION DES FIGURES

D'autres avantages et caractéristiques de l'invention apparaîtront à la lecture de la description suivante donnée à titre d'exemple illustratif et non limitatif, en référence aux figures annexées suivantes :
- la figure 1 représente une vue en perspective en face avant d'un dispositif d'injection manuelle assistée selon un mode de réalisation de l'invention, ainsi qu'un récipient contenant une composition à injecter destiné à être monté de manière fixe sur le dispositif d'injection,
- la figure 2 représente une vue en perspective du dispositif d'injection de la figure 1, le récipient étant monté de manière fixe sur le dispositif d'injection ;
- la figure 3 représente une vue en coupe de côté du dispositif d'injection de la figure 2 ;
- la figure 4A représente une vue en coupe de côté, centrée sur une bague d'adaptation permettant de monter fixement un récipient de faible diamètre sur le dispositif d'injection ;
- la figure 4B représente une vue en coupe de côté similaire à la figure 4A, dans laquelle la bague d'adaptation est retirée afin de permettre à un récipient de grand diamètre d'être monté sur le dispositif d'injection ;
- la figure 5 représente une vue de côté du dispositif d'injection selon un mode de réalisation, qui illustre la prise en main du dispositif selon une prise tridactyle (trois doigts) de type « seringue » ;
- la figure 6 représente une vue de côté du dispositif d'injection selon un mode de réalisation, qui illustre la prise en main du dispositif selon une prise pentadactyle (cinq doigts) de type « seringue » ;
- la figure 7 représente une vue de côté du dispositif d'injection selon un mode de réalisation, qui illustre la prise en main du dispositif selon une prise tridactyle de type « stylo » ;
- la figure 8A est une vue en coupe de côté du dispositif d'injection, qui illustre une étape de mise en place du récipient sur le dispositif d'injection ;
- la figure 8B est une vue en coupe de côté du dispositif d'injection, qui illustre une étape d'accostage du piston par la tige de piston ;
- la figure 8C est une vue en coupe de côté du dispositif d'injection, qui illustre la situation du dispositif d'injection à mi-course de la tige de piston ;
- la figure 8D est une vue en coupe de côté du dispositif d'injection, qui illustre la situation du dispositif d'injection en fin d'injection, la tige de piston étant en fin de course ;
- la figure 8E est une vue en coupe de côté du dispositif d'injection, qui illustre une étape de retrait de la tige de piston en fin d'injection afin de retirer le récipient du dispositif d'injection ;
- la figure 9A est un graphe qui illustre un premier exemple de loi de commande, montrant respectivement l'évolution de la force F2 appliquée par le système d'actionnement sur la tige de piston et la vitesse V de la tige de piston, en fonction de la force F1 appliquée par l'utilisateur sur un capteur du dispositif d'injection ;
- la figure 9B est un graphe qui illustre un premier mode de réalisation d'un deuxième exemple de loi de commande ;
- la figure 9C est un graphe qui illustre un deuxième mode de réalisation d'un deuxième exemple de loi de commande ;
- la figure 9D est un graphe qui illustre un troisième exemple de loi de commande ;
- la figure 9E est un graphe qui illustre un quatrième et un cinquième exemple de loi de commande ;
- la figure 10 est un graphe qui illustre l'évolution de la position axiale de la tige de piston selon l'axe d'injection du dispositif en fonction du temps, lors d'une injection de la composition.

### DESCRIPTION DETAILLEE DE MODES DE REALISATION DE L'INVENTION

L'invention concerne un dispositif d'injection manuelle assistée, qui permet d'injecter une composition contenue dans un récipient.

### Récipient

Le récipient 40, qui peut également être désigné en tant que « contenant primaire », comprend un corps 41 délimité par une surface latérale 42 qui s'étend selon un axe longitudinal A entre une extrémité proximale 43 et une extrémité distale 44 du corps, et définit une chambre apte à contenir une composition à injecter. Le récipient comprend de préférence un bout 45 sur le quel est fixée une aiguille 46. Il s'agit préférentiellement d'un récipient de type prérempli, c'est-à-dire rempli, bouché avec un piston 50, et conditionné avant son utilisation ultérieure pour injection. Alternativement, le récipient peut être initialement vide, puis rempli et bouché juste avant injection. Cette dernière alternative est particulièrement avantageuse lorsque la composition à injecter est obtenue en mélangeant différents composants, par exemple lors de la fabrication d'un ciment osseux à injecter. Après remplissage du récipient avec la composition à injecter, le piston 50 est inséré depuis l'extrémité proximale 43 du récipient et sépare la composition du milieu extérieur. Le récipient est ensuite monté de manière fixe sur le dispositif d'injection de l'invention.

Le piston se trouve au contact de la composition à injecter, et est configuré pour être déplacé grâce au dispositif d'injection, selon un mouvement de translation selon l'axe longitudinal A du récipient en direction distale, afin d'injecter la composition. Cet aspect sera expliqué plus en détail dans la suite du présent texte.

### Dispositif d'injection - structure générale

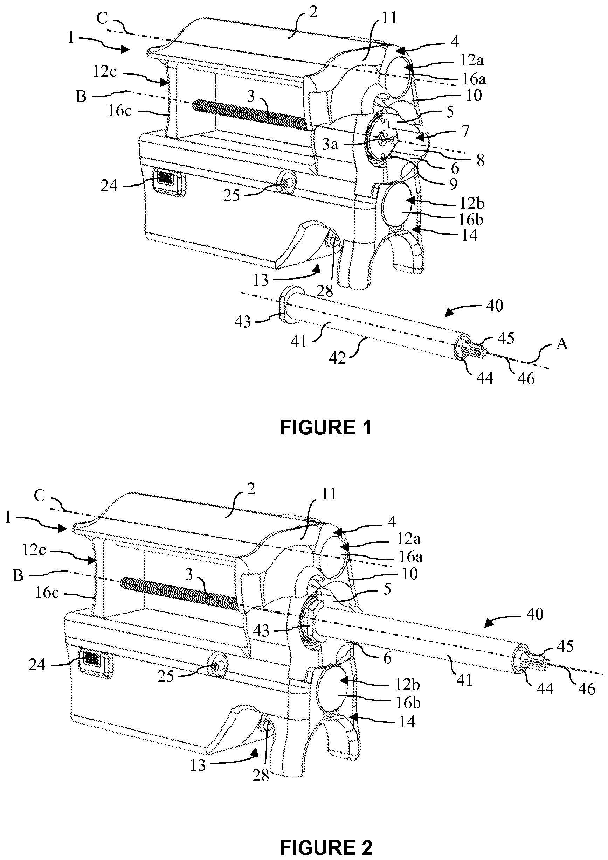

Les figures 1 et 2 illustrent un mode de réalisation du dispositif d'injection 1 selon l'invention.

En référence aux figures 1 et 2, le dispositif d'injection 1 comprend un corps 2 et une tige de piston 3 munie d'une tête 3a à son extrémité distale qui s'étend selon un axe B qui coïncide avec l'axe A du récipient 40 lorsque ce dernier est monté sur le corps du dispositif. La tige de piston 3 comprend de préférence un filetage fonctionnant comme une vis sans fin.

Le dispositif 1 comprend également un système de maintien du récipient configuré pour recevoir et maintenir le récipient de manière fixe par rapport au corps du dispositif. Le récipient 1 est monté de manière amovible sur le système de maintien. Ainsi, l'utilisateur peut changer de récipient pour les différentes injections qu'il doit réaliser.

Sur le dispositif représenté sur la figure 1, le système de maintien comprend un capot 4 au niveau de la partie distale 14 du corps du dispositif, ledit capot délimitant un orifice 7 et étant muni d'une bague de fixation 5 intégrale au capot ou séparable dudit capot. La bague de fixation 5 forme une protrusion 6 qui s'étend en direction distale depuis la surface distale 14 du corps, et qui prolonge l'orifice 7.

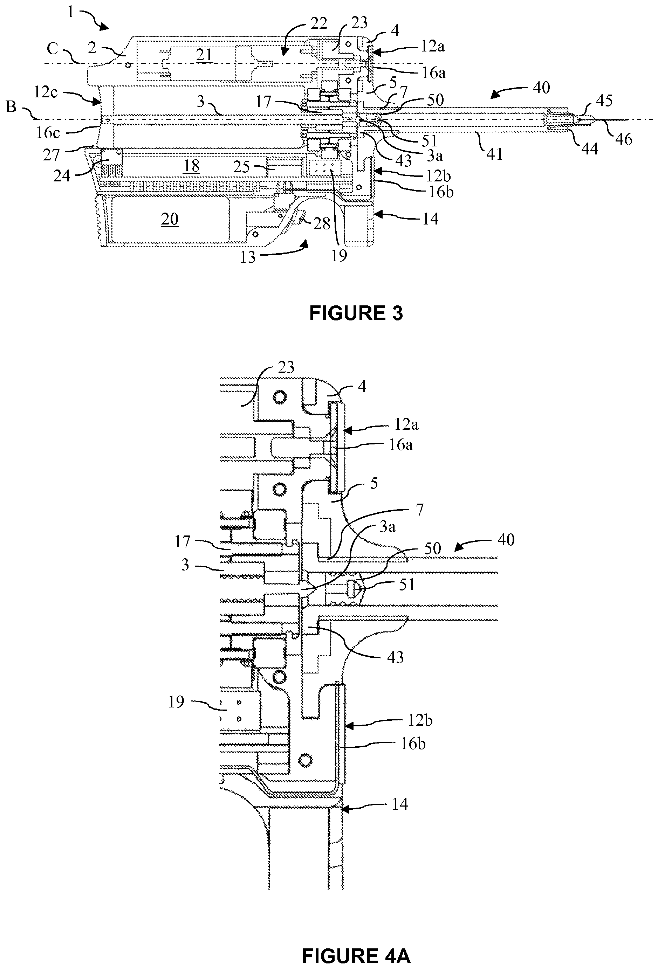

L'extrémité proximale 43 du récipient est insérée dans l'orifice 7 dont le diamètre et la géométrie sont adaptés à cet effet et pour retenir le récipient sur le dispositif pendant son usage. Par exemple, comme l'illustre la figure 3, l'orifice peut présenter une première portion 8 dont le diamètre est légèrement supérieur au diamètre du corps du récipient, et une deuxième portion 9 qui présente un épaulement dont le diamètre est supérieur à celui de la première portion de l'orifice et qui est apte à recevoir la collerette 43 du récipient, une telle collerette étant typiquement présente lorsque le récipient est une seringue comme sur la figure 1. Ainsi, lorsque la seringue est insérée dans l'orifice et traverse l'orifice, le corps de la seringue est positionné dans la première portion 8 de l'orifice et la collerette est positionné dans l'épaulement 9. La seringue est alors bloquée en translation selon l'axe B.

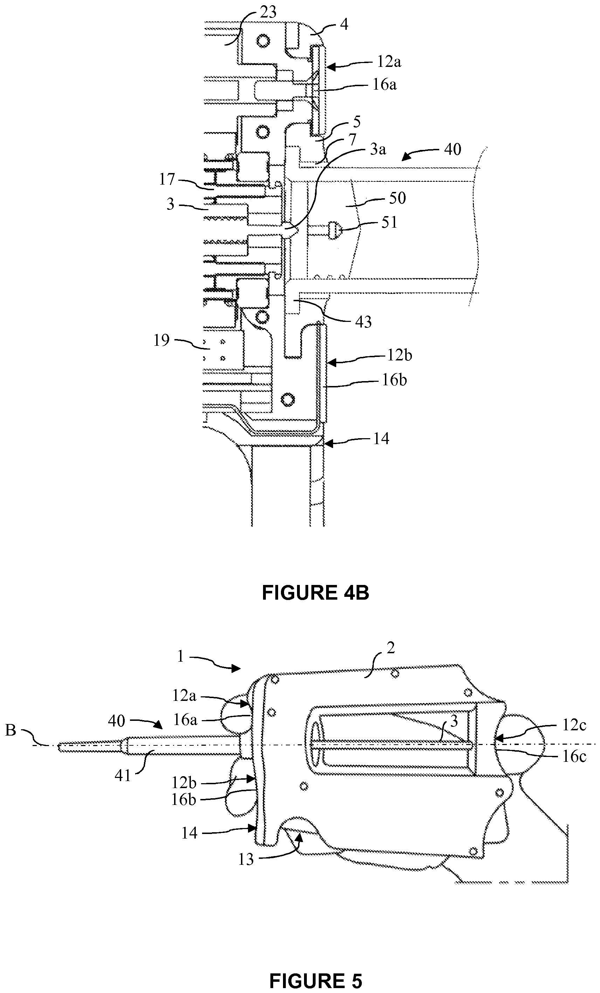

La bague de fixation 5 est ajustée en fonction des dimensions des récipients 40 devant y être montés. Par exemple, sur la figure 4A, la bague de fixation 5 permet comprend un orifice 7 adapté pour insérer une seringue de petit diamètre. Sur la figure 4B, la bague de fixation est absente, l'orifice étant adapté pour insérer une seringue de grand diamètre.

Selon un mode de réalisation préféré, le capot 4 est pivotant. L'une des extrémités du capot est montée en pivot sur le corps du dispositif selon un axe C parallèle à l'axe B, de sorte que le capot s'étend de manière perpendiculaire à l'axe B.

Le capot 4 est mobile en rotation autour de l'axe C, entre une position d'injection dans laquelle l'orifice de la bague est aligné avec l'axe B, et une position libre dans laquelle l'orifice de la bague est désaxé par rapport à l'axe B.

En position libre, l'orifice 7 est accessible par l'utilisateur qui peut alors positionner le récipient dans l'orifice en faisant glisser la bague le long de celui-ci. L'utilisateur rabat ensuite le capot vers le corps du dispositif, jusqu'à la position d'injection.

Selon une variante du mode de réalisation précédent, le capot pivotant 4 est réalisé en plusieurs parties, dont au moins une première partie 10 intégrale avec le corps du dispositif, et au moins une deuxième partie 11 mobile en rotation par rapport à la première partie selon l'axe de rotation C. Les deux parties du système de maintien forment ainsi une pince, dont l'écartement est variable, permettant d'accueillir et d'enserrer le récipient afin de le maintenir fixe pendant l'injection.

Selon cette même variante, la deuxième partie 11 du système de maintien est interchangeable afin de s'adapter à des récipients de différentes dimensions par simple échange de cette deuxième partie par l'utilisateur avec une autre dont l'orifice présente des dimensions adaptées au récipient. Cette deuxième partie interchangeable peut également être codée couleur pour être facilement identifiable par l'utilisateur ou munie d'un dispositif de reconnaissance et de lecture électronique pour indiquer au dispositif les caractéristiques géométriques ou rhéologiques du récipient et de son contenu afin que le dispositif de reconnaissance puisse automatiquement calculer la dose de composition délivrée pour un déplacement unitaire de la tige de piston ainsi que d'autres caractéristiques pour sélectionner rapidement la loi de pilotage.

Le corps 2 du dispositif d'injection est configuré pour être pris en main par l'utilisateur.

A cette fin, le corps 2 du dispositif comprend une ou plusieurs zones de préhension 12a, 12b, 12c au niveau desquelles l'utilisateur peut positionner ses doigts afin de tenir le dispositif en main de manière sûre en évitant que celui-ci ne lui échappe des mains.

Chaque zone de préhension 12a se présente de préférence sous forme d'une surface contre laquelle un ou plusieurs doigt(s) de l'opérateur viennent en appui. Les zones de préhension peuvent alternativement comprendre un évidement 13 dans lequel l'utilisateur peut introduire son doigt afin de rendre la prise plus sûre. Dans ce dernier cas, le doigt de l'utilisateur vient en appui contre la surface interne de l'évidement.

En référence aux figures 1, 2, et 3, le corps du dispositif comprend deux zones de préhension 12a, 12b situées sur la surface distale 14 du corps, et une zone de préhension 12c qui constitue la surface d'appui proximale de la tige de piston 3. L'agencement de ces trois surfaces d'appui selon le mode de réalisation de la figure 1 définit une prise en main du dispositif dite « de type seringue », en ce qu'elle mime la prise en main usuelle à trois doigts d'une seringue : le pouce, l'index, et le majeur.

Selon la prise en main de type seringue, l'utilisateur positionne son index sur la première zone de préhension 12a sur la surface distale 14 du corps, et positionne son majeur et éventuellement son annulaire sur la deuxième zone de préhension 12b. L'utilisateur positionne son pouce sur la troisième zone de préhension 12c constituant la zone d'appui de la tige de piston 3. L'index et le majeur sont ainsi disposés de part et d'autre de l'axe B.

La prise en main de type seringue offre l'avantage d'être familière de l'utilisateur en termes d'ergonomie et de sensations, que celui-ci soit un professionnel médical ou un patient. Par ailleurs, elle offre une bonne perception du déplacement de la tige de piston via le pouce, et donc de la dose injectée lors de l'injection. Elle permet à l'utilisateur d'ajuster facilement la vitesse d'injection en faisant varier l'effort qu'il exerce par son pouce sur la zone d'appui de la tige de piston et concomitamment sur les autres doigts en appui opposé.

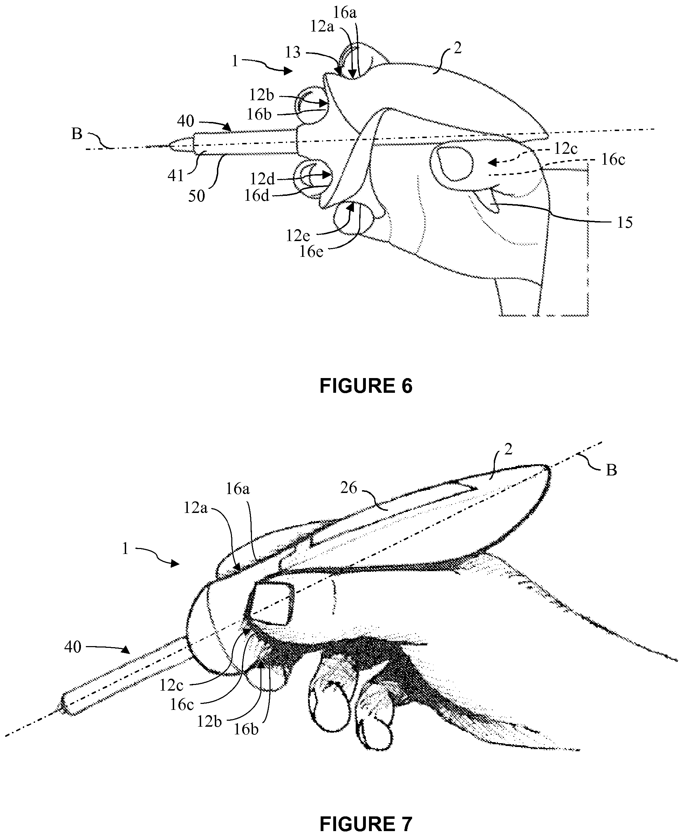

Les figures 5 et 6 illustrent deux modes de réalisation du dispositif d'injection 1 selon l'invention, permettant une prise en main de type seringue.

Sur la figure 5, la prise en main est tridactyle en ce qu'elle fait intervenir trois doigts de l'utilisateur, et de type seringue, de manière similaire au dispositif des figures 1, 2, et 3. L'index est positionné sur la première zone de préhension 12a du corps 2, le majeur est positionné sur la deuxième zone de préhension 12b du corps, et le pouce est positionné sur la troisième zone de préhension 12c constituant la zone d'appui de la tige de piston 3.

Sur la figure 6, la prise en main est une variation du type seringue, et pentadactyle en ce qu'elle fait intervenir cinq doigts de l'utilisateur. L'index, le majeur, l'annulaire, et l'auriculaire sont tous positionnés sur des zones de préhension respectives 12a, 12b, 12d, 12e, situées sur la surface distale 14 du corps. Ces quatre doigts sont ainsi disposés de part et d'autre de l'axe B. Le pouce quant à lui est toujours placé en opposition des quatre autres doigts, mais il est positionné en appui sur une gâchette 15 solidaire de la tige de piston 3, qui s'étend de manière oblique ou perpendiculaire de ladite tige de piston. La force appliquée par l'utilisateur sur la zone de préhension 12c de la gâchette est donc appliquée à la tige de piston 3. Selon ce mode de réalisation la zone de préhension 12c relative associée au pouce est décalée par rapport à la tige de piston 3. Ce décalage permet de réduire l'écartement entre le pouce et les quatre autres doigts par rapport à un positionnement de la troisième zone de préhension à l'extrémité de la tige de piston, tout en conservant la force appliquée par l'utilisateur via son pouce, et concomitamment via les autres doigts en appui opposé, à la tige de piston. Il en résulte une diminution de la fatigue musculaire de la main liée au maintien de la main sur le corps dispositif.

Une autre prise en main possible du dispositif d'injection 1 selon l'invention est la prise dite « de type stylo », en ce qu'elle mime la prise en main usuelle d'un stylo d'écriture. Cette prise en main est illustrée sur la figure 7.

Selon la prise en main de type stylo la plus courante, l'utilisateur positionne son index, son majeur, et son pouce sur trois zones de préhension respectives 12a, 12b, 12c situées autour du corps du dispositif, de préférence sur une portion distale dudit corps. La première 12a et la deuxième zone de préhension 12b, configurées pour recevoir l'index et le majeur, sont situées sensiblement selon une même position axiale selon l'axe longitudinal du corps. La troisième zone de préhension 12c, configurée pour recevoir le pouce, est située de manière proximale par rapport à la première 12a et la deuxième zone de préhension 12b. L'index, le majeur, et le pouce sont ainsi disposés autour de l'axe B.

Sur la figure 7, la prise en main de type stylo illustrée est tridactyle. L'annulaire et le majeur ne sont pas utilisés.

La prise en main de type stylo offre l'avantage de permettre une prise en main ferme et sécurisée du dispositif d'injection, puisque l'index, le majeur et le pouce exercent tous les trois un effort sensiblement perpendiculaire à l'axe longitudinal A et orienté vers le corps du dispositif. De plus, avec ce type de prise en main, la main est plus proche de l'extrémité de l'aiguille, ce qui améliore la précision lors de la piqûre, bien qu'elle ne permette pas de restituer directement à l'utilisateur le déplacement de la tige de piston.

La prise en main du dispositif selon l'invention n'est cependant pas limitée aux prises en main de type seringue et stylo décrites précédemment ou leurs combinaisons, de sorte que le dispositif peut être réalisé et pris en main différemment de ces deux prises en main particulières.

### Capteurs

Le dispositif selon l'invention comprend au moins un capteur de force 16a, 16b, 16c qui permet de connaitre la force, dite également « effort », exercée par l'utilisateur pour réaliser l'injection. Le capteur de force peut être statique ou dynamique.

Un capteur de force statique mesure la force réelle exercée par l'utilisateur. Il peut nécessiter une calibration pour étalonner le signal correspondant à une force considérée comme étant nulle par le capteur, la valeur de la force réellement exercée par l'utilisateur sur le capteur pouvant être nulle ou non nulle.

Un capteur de force dynamique mesure les variations de force. Si une force exercée sur le capteur est maintenue constante pendant une période de temps déterminée, le capteur considère qu'il n'y a plus de force appliquée. Il est donc nécessaire de bien choisir la période de temps en fonction de l'usage prévu. Un avantage de ce type de capteur est qu'il s'auto-étalonne au repos.

Le capteur de force 16a, 16b, 16c est configuré pour recevoir en entrée un effort exercé par l'utilisateur sur ledit capteur. Ce capteur permet, par l'intermédiaire d'un système électromécanique de commande, de piloter un actionneur selon une loi de commande choisie en fonction d'objectifs spécifiques à l'injection de la composition.

Le capteur de force 16a, 16b, 16c est positionné dans une zone de préhension du corps du dispositif d'injection. De cette manière, le capteur de force est directement accessible par l'utilisateur, puisqu'il utilise ses doigts à la fois pour la préhension du dispositif et pour la sollicitation du capteur, et n'a pas besoin d'utiliser d'autres doigts ou bien son autre main pour solliciter le capteur. Le ou les capteurs sont positionnés dans les différentes zones de préhension en fonction du type de prise en main du dispositif, afin d'optimiser l'ergonomie du dispositif.

En référence aux figures 1, 2, et 3, le dispositif d'injection 1 comprend jusqu'à trois capteurs 16a, 16b, 16c positionnés de manière à permettre le fonctionnement du dispositif selon la prise en main de type seringue. Ainsi, un premier capteur 16a est situé dans la première zone de préhension 12a sur la surface distale 14 du corps, un second capteur 16b est situé dans la deuxième zone de préhension 12b sur la surface distale du corps, et un troisième capteur 16c est situé sur la zone d'appui proximale 12c de la tige de piston 3.

De manière alternative, le dispositif peut comprendre seulement un ou deux des trois capteurs susmentionnés.

Selon un mode de réalisation préféré, le dispositif 1 comprend au moins le troisième capteur 16c situé sur la zone d'appui proximale 12c de la tige de piston 3. Ce mode de réalisation offre l'avantage que l'effort exercé par l'utilisateur sur le capteur est indépendant de la répartition de l'effort exercé sur les autres doigts à l'extrémité distale du dispositif. L'effort exercé par l'utilisateur sur le capteur est dirigé dans la même direction que le mouvement de la tige de piston pendant l'injection, c'est-à-dire une translation selon l'axe B en direction distale. Ceci permet à l'utilisateur d'avoir un retour d'information tactile sur la force qu'il exerce sur le capteur en fonction du déplacement de la tige de piston 3 qui en résulte tout au long de l'injection, ainsi que sur la dose injectée via le déplacement de la tige de piston, ce qui améliore ainsi la précision de l'injection.

Bien évidemment, le dispositif 1 peut comprendre seulement le premier 16a et/ou le deuxième capteur 16b sur la surface distale 14 du corps. Dans ce cas, l'effort exercé sur le capteur est parallèle au déplacement de la tige de piston 3, mais orienté en sens contraire c'est-à-dire selon une direction proximale. Ce mode de réalisation fournit également un retour d'information à l'opérateur, mais est cependant mois intuitif.

Selon le mode de réalisation de la figure 6, illustrant la prise en main pentadactyle de type seringue, le dispositif d'injection 1 comprend jusqu'à cinq capteurs 16a, 16b, 16c, 16d, 16e. Ainsi, un capteur 16a est situé dans une des quatre zones de préhension 12a, 12b 12d, 12e sur la surface distale du corps, et un capteur 16c est situé dans la cinquième zone de préhension 12c de la gâchette 15.

Toujours selon la figure 6, le dispositif peut comprendre un capteur 16a par zone de préhension 12a, pour un total de cinq capteurs, ou moins de capteurs que de zones de préhension, par exemple seulement un, deux, ou trois capteurs sur la face distale 14 du corps du dispositif et/ou un capteur sur la gâchette 15.

En référence à la figure 7, le dispositif peut comprendre jusqu'à trois capteurs 16a, 16b, 16c positionnés de manière à permettre le fonctionnement du dispositif selon la prise en main de type stylo. Ainsi, un premier capteur 16a, un deuxième capteur 16b, et un troisième capteur 16c sont situés respectivement dans la première zone de préhension 12a, la deuxième zone de préhension 12b, et la troisième zone de préhension 12c, sur une portion distale du corps du dispositif.

Lorsque le dispositif 1 est conçu pour fonctionner selon la prise en main de type stylo, la présence des trois capteurs 16a, 16b, 16c permet au dispositif de recevoir en entrée trois efforts d'intensités proches dirigés radialement vers le corps du dispositif. Ceci facilite l'utilisation du dispositif en améliorant l'équilibre du corps dans la main de l'utilisateur pendant l'injection, ce qui améliore ainsi grandement la précision de l'injection.

Le dispositif d'injection 1 comprend en outre un système électromécanique, ce dernier comprenant la tige de piston 3, un système d'actionnement 17 de la tige de piston, et un processeur 18. Ces différents éléments sont tout particulièrement visibles sur la figure 3.

Le dispositif d'injection comprend un interrupteur ON/OFF 19 permettant sa mise en fonctionnement ou son arrêt.

Le dispositif d'injection peut comprendre une source d'énergie électrique 20 embarquée pour le fonctionnement du système électromécanique, telle qu'une batterie ou des piles électriques, et fonctionne ainsi de manière autonome. Alternativement, le dispositif d'injection peut être muni d'un câble électrique permettant de le relier à une source d'énergie électrique extérieure ce qui permet de rendre le dispositif plus léger et d'en réduire la taille.

Le système électromécanique comprend un moteur électrique 21 et un réducteur mécanique 22 permettant de réduire la vitesse de rotation du moteur. Un tel réducteur est connu en soi et comprend classiquement des engrenages 23, ces derniers étant agencés à proximité de la surface distale 14 du corps du dispositif de la figure 3.

Le processeur 18 est configuré pour recevoir en entrée un signal de mesure du ou des capteurs 16a, c'est-à-dire un signal représentant une valeur de force exercée par l'utilisateur sur chaque capteur, et fournir en sortie une instruction pour le système d'actionnement 17, qui est fonction du signal du capteur.

A partir de l'instruction du processeur 18, le système d'actionnement convertit la valeur de l'effort mesurée par le capteur 16a (« force d'entrée » ou « effort d'entrée ») en un effort (ou une vitesse) appliqué par la tige de piston 3 (« force de sortie » ou « effort de sortie ») lors de l'utilisation du dispositif, notamment afin d'injecter la composition qui se trouve dans le récipient 40.

Plus il y a de capteurs 16a, 16b, 16c, plus le nombre d'informations en entrée est important, et plus l'effort de sortie est déterminé de manière précise à partir de l'effort d'entrée, par le biais de la loi de commande.

L'effort de sortie peut être en particulier un couple. En effet, selon le mode de réalisation du dispositif illustré sur les figures 1, 2, et 3, la tige de piston 3 comprend un filetage en prise sur un filetage ou un écrou à bille porté sur l'axe d'un engrenage, fonctionnant ainsi comme une vis sans fin. Ainsi, le couple transmis à l'engrenage permet d'entrainer en translation ladite tige de piston qui est bloquée en rotation. Le mouvement de rotation de l'engrenage étant converti en déplacement correspondant en translation de la tige de piston selon l'axe B par le filetage ou l'écrou à bille de l'engrenage d'entraînement.

L'instruction envoyée par le processeur 18 au système d'actionnement 17 est fonction d'une loi de commande programmée et enregistrée dans le processeur. Le pilotage de la tige de piston 3 via la loi de commande peut se faire en force ou bien en vitesse. Lorsque le pilotage se fait en vitesse, le processeur 18 reçoit en entrée le signal de force du capteur 16a et fournit un signal de sortie au système de d'actionnement 17 pour piloter ledit système de d'actionnement selon une certaine vitesse en fonction du signal d'entrée.

Le processeur 18 est avantageusement muni d'une mémoire dans laquelle peuvent être enregistrées des lois de commande et d'autres paramètres de fonctionnement du dispositif.

Par exemple, afin de neutraliser les efforts qui sont nécessaires pour la prise en main du dispositif sans procéder à une injection, la force d'entrée peut être corrigée d'un seuil déterminé, ainsi qu'en linéarité, pour tenir compte des propriété rhéologiques de la composition injectée et de l'aiguille utilisée, ou de la sensibilité recherchée par l'utilisateur en particulier dans la zone de travail pertinente pour l'usage.

La loi de commande peut comprendre différents processus de fonctionnement pour réaliser différentes fonctions correspondantes.

Le dispositif d'injection 1 comprend de préférence un connecteur de programmation 24 pour connecter le dispositif d'injection à un appareil électronique à partir duquel l'utilisateur peut configurer le dispositif d'injection. Un tel appareil électronique peut être par exemple un ordinateur, une tablette électronique, ou encore un téléphone portable. Par l'intermédiaire du connecteur de programmation 24, l'utilisateur peut notamment importer dans la mémoire du dispositif des lois de commande, configurer des lois de commande en fonction de l'utilisation souhaitée, et opérer différents réglages des composants du dispositif d'injection.

Le dispositif d'injection 1 est avantageusement muni d'un moyen de sélection 25, tel qu'un bouton actionnable, permettant de sélectionner une loi de commande parmi celles qui sont enregistrées dans la mémoire, afin que celle-ci soit mise en oeuvre lors de l'utilisation du dispositif.

Le dispositif d'injection 1 est avantageusement muni d'un moyen d'ajustement, tel qu'un bouton actionnable, permettant d'ajuster le seuil F1s de déclenchement de l'assistance par loi de commande, c'est-à-dire la valeur de l'effort d'entrée à partir de laquelle s'opère le déclenchement de la loi de commande choisie. Ainsi, tant que l'effort d'entrée est inférieur au seuil de déclenchement F1s, l'assistance par loi de commande n'est pas déclenchée. La valeur de l'effort d'entrée correspond à la valeur de l'effort de sortie. Lorsque l'effort d'entrée devient supérieur ou égal au seuil de déclenchement F1s, l'assistance par loi de commande se déclenche et vient assister l'utilisateur dans l'utilisation du dispositif. Afin de simplifier l'utilisation du dispositif, un unique moyen 25 peut permettre à la fois d'ajuster le seuil F1s de déclenchement de l'assistance et de sélectionner une loi de commande, comme c'est le cas pour le dispositif illustré sur les figures 1, 2, et 3.

Le dispositif d'injection 1 peut être muni d'une interface utilisateur, comprenant un écran 26 tel qu'illustré sur la figure 7. Cette interface permet à l'utilisateur d'enregistrer et de sélectionner les paramètres de fonctionnement du dispositif, par exemple les lois de commande, le type de récipient utilisé, ou encore le volume et le type de composition à injecter, et d'avoir un retour d'information du dispositif, notamment visuel via l'écran, sur lesdits paramètres de fonctionnement lors de l'utilisation du dispositif. L'utilisateur peut également choisir et/ou être informé des lois de commande enregistrées dans la mémoire, la loi de commande choisie actuellement, le seuil de déclenchement de l'assistance, le témoin de charge de la batterie, le volume de composition injectée depuis la dernière mise en marche du dispositif, ou depuis la dernière injection de composition, ou depuis la dernière loi de commande sélectionnée. D'autres paramètres, notamment des informations variables dans un intervalle de temps de l'ordre de la durée d'une injection (quelques secondes à quelques minutes) peuvent également être fournies à l'utilisateur via l'interface, tels que l'effort d'entrée, l'effort de sortie, et la vitesse de la tige de piston c'est-à-dire la vitesse d'injection.

### Procédé de fonctionnement

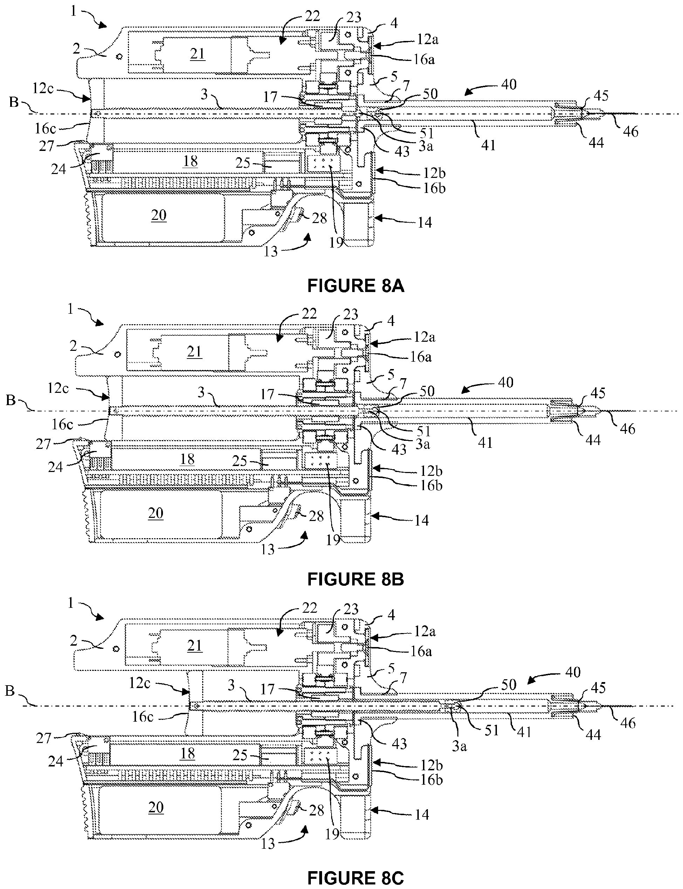

Le fonctionnement du dispositif d'injection 1 va maintenant être décrit en référence à la figure 2 et aux figures 8A-E, pour illustrer l'injection d'une composition contenue dans un récipient 40 de type PFS.

En référence à la figure 2, la seringue 40 est montée sur le dispositif d'injection 1. Le capot pivotant 4 est ouvert, c'est-à-dire que la deuxième partie 11 du capot est écartée de la première partie 10. La seringue 40 est déplacée de manière perpendiculaire à l'axe B, de façon à insérer la collerette 43 dans l'épaulement 9 de la bague de fixation 5.

La deuxième partie 11 du capot est ensuite réunie avec la première partie 10, afin d'enserrer la partie proximale de la seringue 40 et de maintenir ladite seringue de manière fixe et dans l'alignement de la course de la tige de piston. Le dispositif d'injection est alors tel que représenté sur la figure 8A.

La zone de préhension 12c de la tige de piston 3 est au contact d'un élément de butée proximale 27 qui délimite la fin de course proximale de la tige de piston. En d'autres termes, la tige de piston 3 ne peut pas se trouver dans une position davantage proximale que celle de la figure 8A. Cette position dite « position de démarrage » est détectée par le processeur 18.

En référence à la figure 8B, le système électromécanique lance de préférence une étape automatisée d'accostage du piston 50 par la tige de piston 3. La tige de piston se déplace donc automatiquement, sans intervention de l'utilisateur sur la tige de piston, en direction distale jusqu'à venir en appui contre le piston 50.

A la fin de l'étape d'accostage, l'extrémité distale ou tête 3a de la tige de piston 3 est insérée dans le piston 50, ce dernier étant avantageusement muni d'un évidement 51 dont la forme correspond à celle de l'extrémité distale 3a de la tige de piston afin d'assurer un meilleur contact entre la tige de piston et le piston, ce qui améliore la précision de l'injection. De plus, la zone de préhension 12c de la tige de piston se trouve à une distance déterminée de l'élément de butée proximale 27. Cette distance est détectée par le processeur. La tige de piston se trouve alors dans une position dite « position de départ », et prête à être actionnée.

L'utilisateur peut alors procéder à l'injection de la composition, en exerçant une force sur un ou plusieurs capteurs 16a, 16b, 16c du dispositif. La figure 8C illustre la situation à mi-course distale de la tige de piston.

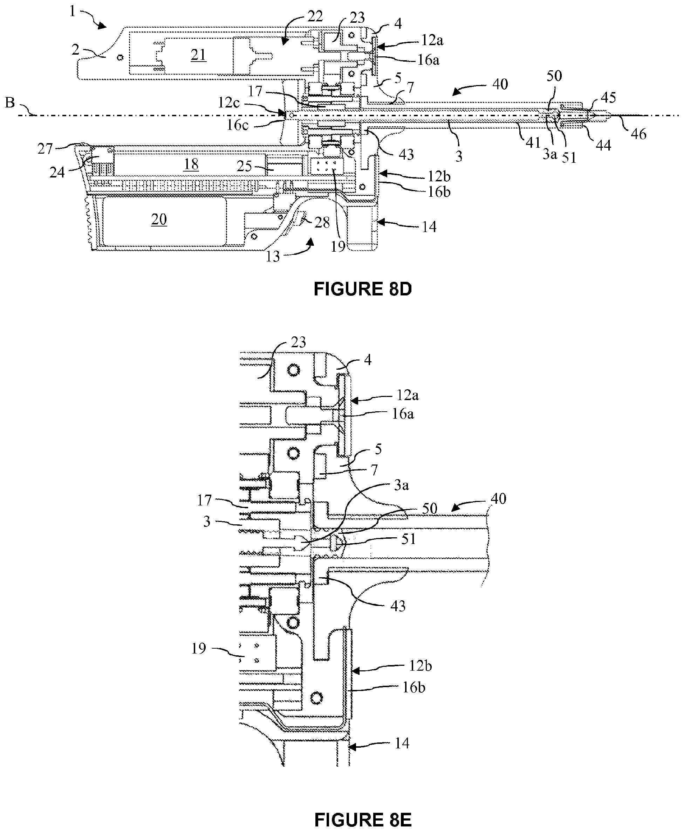

La tige de piston 3 se déplace en direction distale dans le récipient 40, entrainant avec lui le piston 50, au fur et à mesure de l'injection de la composition. La figure 8D illustre la situation en fin de course distale de la tige de piston. Toute la composition a été injectée, et le piston 50 est en butée contre l'extrémité distale du récipient.

En référence à la figure 8E, le système électromécanique lance de préférence une étape automatisée de désaccostage de la tige de piston 3. La tige de piston se déplace donc automatiquement, sans intervention de l'utilisateur sur la tige de piston, en direction proximale jusqu'à ce que la zone de préhension 12c de la tige de piston vienne au contact de l'élément de butée proximale 27.

La tige de piston se trouve à l'extérieur du récipient, ce dernier pouvant alors être retiré du système de maintien du dispositif. L'utilisateur peut ensuite procéder au chargement d'un nouveau récipient et procéder à une autre injection ou bien arrêter le dispositif.

### Exemple de lois de commande

Des exemples de processus de fonctionnement correspondant à des lois de commande vont maintenant être décrits selon plusieurs modes de réalisation. Les modes de réalisation sont combinables entre eux, de sorte que le dispositif peut être configuré pour fonctionner selon un ou plusieurs des processus de fonctionnement décrits.

### Loi simple

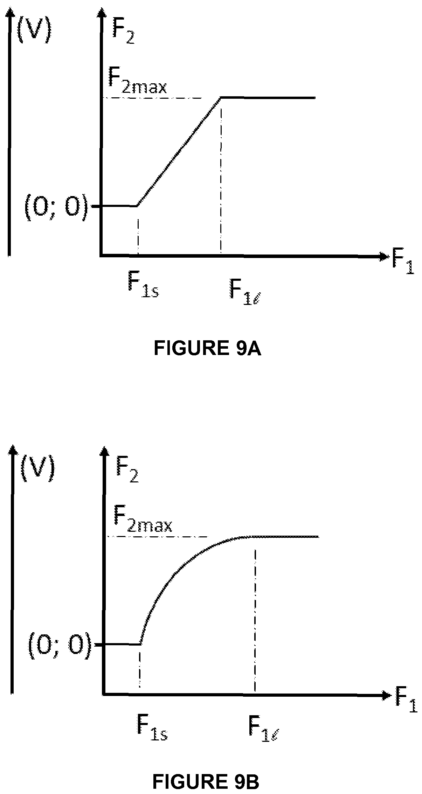

Selon un premier exemple illustré sur la figure 9A, la loi de commande est une loi d'injection simple selon laquelle l'effort de sortie F2 est sensiblement proportionnel à l'effort d'entrée F1.

Etant donné que le dispositif d'injection selon l'invention est également pilotable en vitesse, la vitesse fournie par le système d'actionnement en sortie est mesurée en ordonnée, en parallèle de l'effort de sortie F2. Les observations faites au regard de l'effort de sortie F2 et de la vitesse de sortie F2 sont les mêmes.

L'effort de sortie F2 reste nul jusqu'à ce que l'effort d'entrée F1 exercé par l'utilisateur sur le ou les capteurs atteigne le seuil de déclenchement F1s à partir duquel l'assistance par loi de commande se déclenche. L'effort de sortie F2 augmente alors avec l'effort d'entrée F1.

Sur la figure 9A, l'augmentation de F2 avec F1 est représentée par une droite et correspond à une fonction linéaire. Cette représentation est simplifiée. En pratique, le comportement dispositif dépend notamment du type de seringue, du taux de remplissage de la seringue, et de la viscosité de la composition, de sorte que l'augmentation de F2 avec F1 se traduit plutôt par une courbe.

L'effort de sortie F2 augmente jusqu'à un plafond F2max lorsque l'effort d'entrée atteint F1ℓ. Cette valeur F2max est enregistrée dans la mémoire du dispositif d'injection notamment lors de sa fabrication, et permet d'éviter tout risque d'explosion de la seringue ou éjection intempestive de l'aiguille dus à un effort de sortie F2 trop élevé.

### Multiplication d'effort

Selon un deuxième exemple, la loi de commande comprend un processus de multiplication d'effort. Selon ce processus, l'effort de sortie F2 (respectivement la vitesse de sortie) du système d'actionnement est augmenté principalement selon un multiple de la valeur de l'effort d'entrée F1.

A cet effet, le système d'actionnement 17 comprend un système de multiplication d'effort configuré pour fournir un effort de sortie F2 pour l'actionnement de la tige de piston 3 qui est un multiple de la valeur de l'effort F1 reçu en entrée par le processeur 18.

La figure 9B illustre un premier mode de réalisation d'une loi de commande à multiplication d'effort. L'effort de sortie F2 reste nul jusqu'à ce que l'effort d'entrée F1 exercé par l'utilisateur sur le ou les capteurs 16a, 16b, 16c atteigne le seuil de déclenchement F1s à partir duquel l'assistance par loi de commande se déclenche. L'effort de sortie F2 augmente ensuite très fortement, de manière asymptotique, puis se stabilise à F2max lorsque l'effort d'entrée F1 atteint un plafond F1ℓ. L'injection est donc grandement facilitée pour les faibles valeurs de F1 < F1ℓ. Le dispositif d'injection est très sensible à partir du seul F1s et peu sensible après le plafond F1ℓ.

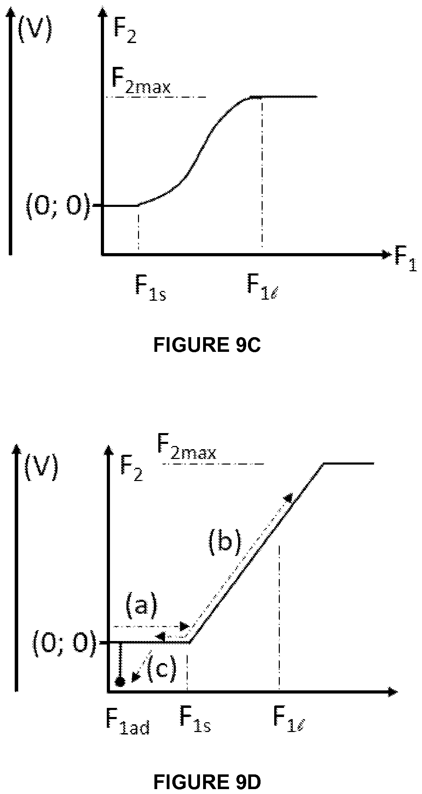

La figure 9C illustre un deuxième mode de réalisation d'une loi de commande à multiplication d'effort. L'effort de sortie F2 reste nul jusqu'à ce que l'effort d'entrée F1 exercé par l'utilisateur sur le ou les capteurs 16a, 16b, 16c atteigne le seuil de déclenchement F1s à partir duquel l'assistance par loi de commande se déclenche. L'effort de sortie F2 augmente ensuite doucement puis fortement, jusqu'à se stabiliser à F2max lorsque l'effort d'entrée F1 atteint le plafond F1ℓ. Le déclenchement de l'assistance est donc plus progressif que pour le premier mode de réalisation en offrant une grande plage de sensibilité en milieu de plage de fonctionnement.

### Dispositif anti-goutte

Dans les dispositifs d'injection de l'état de l'art, la pression interne dans le récipient et la déformation dans le système d'actionnement de la tige de piston lors de l'injection de produits visqueux résultent en un reliquat d'injection de la composition alors que l'opérateur a cessé d'appuyer sur la tige de piston de la seringue, par un mécanisme de détente de la pression interne accumulée. Ce surplus de composition injectée, qui n'est pas souhaité, est problématique dans le cas où une dose très précise de composition est requise ou que l'utilisateur souhaite déplacer rapidement l'aiguille alors qu'il ne faut pas déposer de matière parasite lors du retrait de l'aiguille, comme c'est le cas pour certains ciments osseux qui polymérisent rapidement utilisés en vertébroplastie et qui ne doivent pas être déposés hors de la vertèbre traitée.

Afin de pallier cet inconvénient, selon un troisième exemple, la loi de commande comprend un processus qui permet d'opérer un retour arrière de la tige de piston 3, c'est-à-dire un déplacement de la tige de piston dans la direction proximale par rapport au corps 2 du dispositif selon une distance déterminée, lorsque l'utilisateur souhaite arrêter l'injection, soit de manière définitive par exemple lorsque toute la composition a été injectée et que la tige de piston 3 est en fin de course, soit de manière ponctuelle par exemple lorsque l'opérateur réalise une série d'injections en marquant un temps d'arrêt entre deux injections consécutives.

Ce processus permet ainsi de supprimer tout surplus de composition, et d'injecter ainsi une dose précise de composition en des lieux choisis, sans bavures.

Lorsque l'injection est interrompue, momentanément ou définitivement, l'effort F1 exercé par l'utilisateur sur le capteur 16a, 16b, 16c diminue en-dessous d'un seuil de retrait déterminé F1ad, égal ou inférieur au seuil d'activation F1s, sans devenir nul. La valeur de l'effort F1 du signal reçu par le processeur 18 en provenance du capteur diminue alors que le dispositif d'injection est en fonctionnement. Dans ce cas, le processeur 18 est configuré pour envoyer une instruction au système d'actionnement 17 qui déplace la tige de piston 3 en retour arrière dès que l'effort d'entrée F1 devient inférieur ou égal au seuil F1ad. Le seuil de retrait F1ad est ajustable en fonction du type d'injection réalisée par l'utilisateur. Lorsque l'injection nécessite une très grande précision, le seuil F1ad peut être élevé et proche du seuil F1s de déclenchement de l'assistance. Dans ce cas, un léger relâchement de l'utilisateur permet d'arrêter l'injection et d'activer le retour arrière. En revanche, lorsqu'une précision moins élevée est requise, le seuil F1ad peut être plus faible, voire nul. Dans ce cas, l'arrêt de l'injection et l'activation du retour arrière ne sont effectifs que lorsque l'utilisateur relâche totalement la pression exercée sur le capteur.

Afin d'éviter tout retour arrière intempestif, une valeur de temps est avantageusement enregistrée dans la mémoire du processeur afin que le retour arrière ne soit opéré que lorsque l'intervalle de temps Δt écoulé à partir de la fin de l'injection soit égal à cette valeur de temps. Le processeur est avantageusement muni d'une horloge programmée pour mesurer l'intervalle de temps Δt.

La figure 9D illustre un troisième exemple d'une loi de commande pour processus anti-goutte. (a) L'effort de sortie F2 reste nul jusqu'à ce que l'effort d'entrée F1 exercé par l'utilisateur sur le ou les capteurs 16a, 16b, 16c atteigne le seuil de déclenchement F1s à partir duquel l'assistance par loi de commande se déclenche ; (b) l'effort de sortie F2 augmente au fur et à mesure de l'injection ; (c) l'effort de sortie F2 diminue avec la diminution de l'effort d'entrée F1 après injection jusqu'à s'annuler (retour arrière le long de la courbe) ; (d) l'effort de sortie F2 diminue encore et passe en-dessous de la valeur nulle de départ lorsque F1 atteint la valeur F1ad, ce qui traduit un retour de la tige de piston en direction proximale d'une distance prédéterminée réglable, et l'aspiration de la goutte.

L'effort d'entrée F1 n'est pas tout à fait nul, puisque l'utilisateur tient toujours le dispositif en main et donc exerce un effort, même faible, sur le capteur. Tant que l'effort d'entrée est compris entre F1ad et F1s, l'assistance reste désactivée.

### Détection de sang

Lors de l'injection d'une composition dans des tissus irrigués, il est possible que l'aiguille transperce un vaisseau sanguin. Les dispositifs d'injection de l'état de l'art n'offrent aucune possibilité de détecter la pénétration d'un vaisseau sanguin par l'aiguille.

Afin de pallier cet inconvénient, selon un quatrième exemple, la loi de commande comprend un processus qui permet de réaliser une aspiration calibrée afin de détecter l'aspiration de sang par l'extrémité de l'aiguille qui reflue à l'extrémité proximale de l'aiguille. Si l'utilisateur constate qu'il n'y a pas de sang aspiré, il peut poursuivre l'injection. Dans le cas contraire, il déplace l'aiguille dans les tissus. Le déclenchent par l'utilisateur de la procédure de détection de sang peut être fait par un bouton poussoir dédié ou en utilisant l'information temporelle d'un des capteurs de force, par exemple au travers d'un double appui rapide.

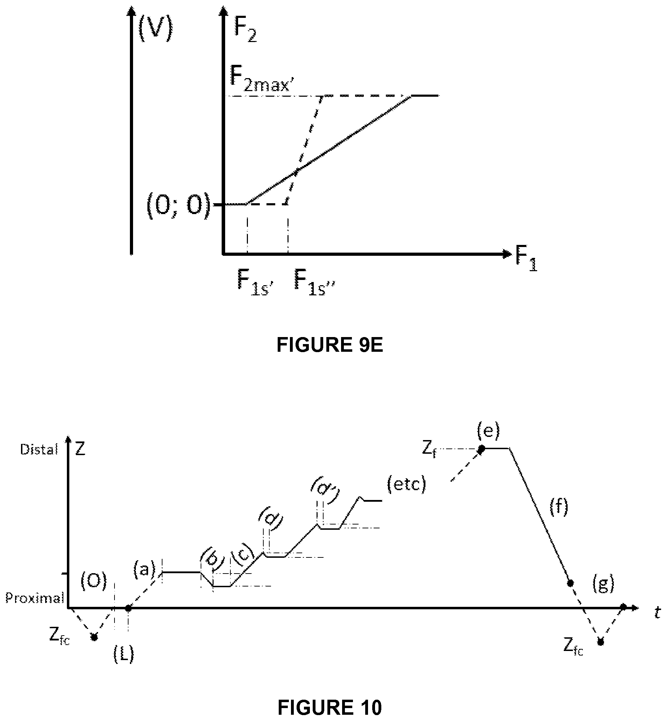

La figure 9E illustre un quatrième et un cinquième exemple de loi de commande. Ces exemples traduisent le fait que le seuil d'activation F1s est réglable (valeur F1s' ou F1s"), notamment en fonction de la seringue et de l'utilisation du dispositif d'injection. Bien que ceci ne soit pas représenté, le plafond F1ℓ, le plafond F2max, ainsi que le seuil F1 sont également réglables.

La figure 10 est un graphe qui illustre l'évolution de la position axiale Z de la tige de piston selon l'axe d'injection du dispositif en fonction du temps t, lors d'une série de d'injections d'une même composition.
(a) Le dispositif est mis en fonctionnement. La tige de piston 3 recherche sa position de démarrage et se déplace donc en direction distale jusqu'à venir au contact de l'élément de butée proximale 27. Sur le graphe, cela se traduit par une diminution de Z en fonction de t. Le système électromécanique lance ensuite une étape automatisée d'accostage du piston 50 par la tige de piston 3. La tige de piston se déplace donc automatiquement en direction distale jusqu'à venir en appui contre le piston. Sur le graphe, cela se traduit par une augmentation de Z.
(b) Le dispositif effectue ensuite un processus de détection d'un vaisseau sanguin sur déclenchement de l'utilisateur. L'aspiration calibrée et le déplacement arrière de la tige de piston 3 se traduit par une faible diminution de Z. La courbe est ensuite plane pendant une durée déterminée, ce qui correspond à une phase d'attente au cours de laquelle aucun effort n'est exercé sur le capteur 16a, 16b, 16c.
(c) L'injection est ensuite réalisée. Le piston 50 se déplace en direction distale suite à un effort exercé par l'utilisateur sur le capteur, et une dose correspondante de composition est injectée. Z augmente alors en fonction de t.
(d) Le dispositif effectue ensuite un processus anti-goutte. Le retour arrière de la tige de piston 3 se traduit par une très faible diminution de Z.
   La série d'injections est ensuite poursuivi, chaque injection (c'), (c"), etc... étant préférentiellement suivie d'un processus anti-goutte (d'), (d"), etc... et d'une phase d'attente.
(e) En fin de course de la tige de piston, le dispositif lance automatiquement une étape de retour arrière de la tige de piston 3 de désaccostage de la tige de piston du piston 50. La tige de piston se déplace donc automatiquement, sans intervention de l'utilisateur sur la tige de piston, en direction proximale jusqu'à ce que la zone de préhension de la tige de piston vienne au contact de l'élément de butée proximale 27.

## Revendications

1. Dispositif (1) d'injection manuelle assistée pour injecter une composition contenue dans un récipient (40), dans lequel le récipient (40) est muni à son extrémité distale d'une aiguille (46) et contient un piston (50), le dispositif d'injection comprenant :
- un corps (2) comprenant au moins une zone de préhension (12a) par laquelle le corps peut être pris en main par un utilisateur,
- un élément de maintien (4) du récipient configuré pour recevoir et maintenir le récipient (40) de manière fixe par rapport au corps,
- un système électromécanique comprenant :
• une tige de piston (3) apte à se déplacer en translation dans le récipient (40) par rapport au corps (2) du dispositif afin de déplacer le piston (50),
• un système d'actionnement (17) de la tige de piston (3),
• un processeur (18) apte à communiquer avec le système d'actionnement (17),
le dispositif (1) étant **caractérisé en ce qu'**il comprend au moins un capteur de force (16a) positionné sur le corps au niveau d'une zone de préhension, le capteur de force étant configuré pour recevoir en entrée un effort mécanique F1 exercé sur ledit capteur (16a) par l'utilisateur lors de l'injection de la composition, et pour transmettre au processeur (18) un signal de sortie F2 qui est fonction dudit effort mécanique,
et le processeur (18) est configuré pour envoyer une instruction au système d'actionnement (17) en fonction du signal de sortie du capteur (16a) pour actionner la tige de piston (3) selon une loi de commande prédéterminée, afin d'ajuster l'effort mécanique devant être exercé par l'utilisateur sur la tige de piston (3) pour injecter la composition.

2. Dispositif (1) selon la revendication 1, dans lequel le système d'actionnement (17) comprend un système de multiplication d'effort configuré pour fournir à la tige de piston (3) un effort mécanique F2 qui est un multiple de l'effort F1 exercé par l'utilisateur sur le capteur (16a), et dans lequel la loi de commande est une loi de multiplication d'effort.

3. Dispositif (1) selon la revendication 1 ou la revendication 2, dans lequel la loi de commande définit un déplacement de la tige de piston (3) dans une direction proximale selon une distance D1 prédéterminée lorsque l'injection est interrompue et que l'effort mécanique F1 exercé par l'utilisateur sur le capteur (16a) est inférieur à une valeur seuil F1ad déterminée pendant une durée déterminée Δt1.

4. Dispositif (1) selon l'une des revendications précédentes, dans lequel la tige de piston (3) présente une position de démarrage, et la loi de commande définit un déplacement de la tige de de piston dans une direction proximale selon une distance D2 prédéterminée lorsque l'injection est terminée et que l'effort mécanique F1 exercé par l'utilisateur sur le capteur (16a) est inférieur à une valeur seuil F1ad déterminée pendant une durée déterminée Δt2 ou lorsque la tige de piston est en fin de course distale.

5. Dispositif (1) selon l'une des revendications précédentes, dans lequel l'aiguille (46) étant positionnée dans une zone sous-cutanée du corps humain, la loi de commande définit un déplacement de la tige de piston (3) dans une direction proximale selon une distance D3 prédéterminée afin de réaliser une aspiration calibrée d'un volume déterminé d'un fluide humain, afin de détecter la présence éventuelle de sang dans l'aiguille.

6. Dispositif (1) selon l'une des revendications précédentes, comprenant un moyen d'activation sélectif (25) permettant à l'utilisateur de sélectivement activer ou désactiver le pilotage de la tige de piston (3) par le processeur (18) via la loi de commande.

7. Dispositif (1) selon la revendication 6, dans lequel le moyen d'activation sélectif (25) comprend un moyen de réglage permettant à l'utilisateur de choisir un seuil F1s de déclenchement, de sorte que le pilotage de la tige de piston (3) par le processeur (18) via la loi de commande se déclenche seulement lorsque le l'effort F1 exercé par l'utilisateur sur le capteur (16a) est supérieur ou égal au seuil F1s de déclenchement.

8. Dispositif (1) selon l'une des revendications précédentes, dans lequel le corps (2) du dispositif d'injection comprend au moins deux zones de préhension (12a, 12b) sur une surface distale (14) du corps du dispositif, dont au moins une des zones de préhension (12a) est munie d'un capteur (16a) et est apte à recevoir l'appui de l'index, du majeur, de l'annulaire, ou de l'auriculaire de l'utilisateur, et la tige de piston (3a) comprend une extrémité proximale comprenant une zone de préhension (12c), ladite zone de préhension (12c) étant munie d'un capteur (16c) et étant apte à recevoir l'appui du pouce de l'utilisateur, pour permettre une prise en main du dispositif de type seringue.

9. Dispositif (1) selon la revendication 8, dans lequel la zone de préhension (12c) de la tige de piston (3) est positionnée sur une gâchette (15) solidaire de la tige de piston et qui s'étend de manière oblique ou perpendiculaire à la tige de piston depuis une position distale par rapport à l'extrémité proximale de ladite tige de piston (3).

10. Dispositif (1) selon l'une des revendications 1 à 7, comprenant au moins trois zones de préhension (12a, 12b, 12c) agencées sur le corps (2) du dispositif autour d'un axe longitudinal (B) du dispositif, dont au moins une des zones de préhension (12a) est munie d'un capteur (16a), lesdites zones de préhension (12a, 12b, 12c) étant aptes à recevoir respectivement l'appui de l'index, du majeur, ou du pouce de l'utilisateur pour permettre une prise en main du dispositif de type stylo.

11. Dispositif (1) selon l'une des revendications précédentes, dans lequel l'élément de maintien du récipient (40) comprend un capot pivotant (4) muni d'un orifice (7) apte à recevoir au moins l'extrémité proximale (43) du récipient, le capot pivotant (4) étant apte à pivoter entre une position d'injection dans laquelle l'orifice (7) est aligné avec la course de la tige de piston (3), et une position libre dans laquelle l'orifice (7) est désaxé par rapport à la course de la tige de piston (3) et accessible par l'utilisateur pour permettre l'insertion du récipient (50) dans ledit orifice.

12. Dispositif (1) selon l'une des revendications précédentes, dans lequel l'élément de maintien du récipient (40) comprend en outre une pluralité de bagues (5) interchangeables munies d'orifices (7) de différents diamètres adaptés pour recevoir des récipients (40) correspondants de différents diamètres.

13. Dispositif (1) selon la revendication 12, dans lequel le capot pivotant (4) est réalisé en plusieurs parties, dont au moins une première partie (10) intégrale avec le corps (2) du dispositif, et au moins une deuxième partie (11) mobile apte à pivoter entre la position d'injection et la position libre, les deux parties (10, 11) du capot pivotant (4) formant ainsi une pince à écartement variable permettant d'accueillir et d'enserrer le récipient (40) afin de le maintenir en position fixe.

14. Dispositif (1) selon la revendication 13, dans lequel la deuxième partie (11) mobile est apte à être détachée du corps (2) du dispositif.

15. Dispositif (1) selon la revendication 14, dans lequel la deuxième partie (11) mobile est codée couleur pour être identifiable par l'utilisateur ou est munie d'un dispositif de reconnaissance et de lecture électronique pour indiquer au processeur (18) les caractéristiques géométriques ou rhéologiques du récipient (40) et de son contenu afin que le dispositif de reconnaissance puisse automatiquement calculer la dose de composition délivrée pour un déplacement unitaire de la tige de piston (3).

16. Dispositif (1) selon l'une des revendications précédentes, dans lequel le système électromécanique comprend une mémoire dans laquelle sont enregistrées des lois de commande et/ou des valeurs seuils.

17. Dispositif (1) selon l'une des revendications précédentes, comprenant en outre une interface utilisateur (26) permettant à l'utilisateur d'enregistrer et de sélectionner les paramètres de fonctionnement du dispositif, et d'avoir un retour d'information du dispositif sur lesdits paramètres de fonctionnement lors de l'utilisation du dispositif.

## Patentansprüche

1. Handunterstützte Injektionsvorrichtung (1) zum Injizieren einer in einem Behälter (40) enthaltenen Zusammensetzung, wobei der Behälter (40) an seinem distalen Ende mit einer Nadel (46) versehen ist und einen Kolben (50) enthält, die Injektionsvorrichtung umfassend:
- einen Körper (2), der mindestens einen Griffbereich (12a) umfasst, durch den der Körper von einem Benutzer gefasst werden kann,
- ein Halteelement (4) des Behälters, das so eingerichtet ist, dass es den Behälter (40) fest in Bezug auf den Körper aufnimmt und hält,
- ein elektromechanisches System, umfassend:
• eine Kolbenstange (3), die sich in dem Behälter (40) relativ zu dem Körper (2) der Vorrichtung translatorisch verschieben kann, um den Kolben (50) zu bewegen,
• ein Betätigungssystem (17) der Kolbenstange (3),
• einen Prozessor (18), der mit dem Betätigungssystem (17) kommunizieren kann,
wobei die Vorrichtung (1) **dadurch gekennzeichnet ist, dass** sie mindestens einen Kraftsensor (16a) umfasst, der auf dem Körper in Höhe einer Greifzone positioniert ist, wobei der Kraftsensor so konfiguriert ist, dass er als Eingang eine mechanische Kraft F1 empfängt, die vom Benutzer bei der Injektion der Zusammensetzung auf den Sensor (16a) ausgeübt wird, und dass er dem Prozessor (18) ein Ausgangssignal F2 übermittelt, das von der mechanischen Kraft abhängig ist, und der Prozessor (18) so konfiguriert ist, dass er in Abhängigkeit von dem Ausgangssignal des Sensors (16a) eine Anweisung an das Betätigungssystem (17) sendet, um die Kolbenstange (3) gemäß einem vorbestimmten Steuergesetz zu betätigen, um die mechanische Kraft anzupassen, die von dem Benutzer auf die Kolbenstange (3) zum Injizieren der Zusammensetzung ausgeübt werden muss.

2. Vorrichtung (1) nach Anspruch 1, wobei das Betätigungssystem (17) ein Kraftvervielfältigungssystem umfasst, das so konfiguriert ist, dass es der Kolbenstange (3) eine mechanische Kraft F2 bereitstellt, die ein Vielfaches der vom Benutzer auf den Sensor (16a) ausgeübten Kraft F1 ist, und wobei das Steuergesetz ein Kraftvervielfältigungsgesetz ist.

3. Vorrichtung (1) nach Anspruch 1 oder 2, wobei das Steuergesetz eine Verschiebung der Kolbenstange (3) in einer proximalen Richtung um einen vorbestimmten Abstand D1 festlegt, wenn die Injektion unterbrochen wird und die vom Benutzer auf den Sensor (16a) ausgeübte mechanische Kraft F1 für eine bestimmte Dauer Δt1 unter einem bestimmten Schwellenwert F1ad liegt.

4. Vorrichtung (1) nach einem der vorhergehenden Ansprüche, wobei die Kolbenstange (3) eine Startposition aufweist und das Steuergesetz eine Verschiebung der Kolbenstange in proximaler Richtung um eine vorbestimmte Strecke D2 festlegt, wenn die Injektion beendet ist und die vom Benutzer auf den Sensor (16a) ausgeübte mechanische Kraft F1 für eine bestimmte Zeit Δt2 unter einem bestimmten Schwellenwert F1ad liegt oder wenn die Kolbenstange am Ende ihres distalen Hubs ist.

5. Vorrichtung (1) nach einem der vorhergehenden Ansprüche, bei der die Nadel (46) in einem subkutanen Bereich des menschlichen Körpers positioniert ist, wobei das Steuergesetz eine Verschiebung der Kolbenstange (3) in einer proximalen Richtung um einen vorbestimmten Abstand D3 festlegt, um eine kalibrierte Absaugung eines bestimmten Volumens einer menschlichen Flüssigkeit durchzuführen, um das mögliche Vorhandensein von Blut in der Nadel zu erkennen.

6. Vorrichtung (1) nach einem der vorhergehenden Ansprüche, die ein selektives Aktivierungsmittel (25) umfasst, das es dem Benutzer ermöglicht, die Steuerung der Kolbenstange (3) durch den Prozessor (18) über das Steuergesetz selektiv zu aktivieren oder zu deaktivieren.

7. Vorrichtung (1) nach Anspruch 6, bei der das Mittel zur selektiven Aktivierung (25) ein Einstellmittel umfasst, das es dem Benutzer ermöglicht, eine Schwelle F1s für die Auslösung zu wählen, so dass die Steuerung der Kolbenstange (3) durch den Prozessor (18) über das Steuergesetz nur dann ausgelöst wird, wenn die vom Benutzer auf den Sensor (16a) ausgeübte Kraft F1 größer oder gleich der Schwelle F1s für die Auslösung ist.

8. Vorrichtung (1) nach einem der vorhergehenden Ansprüche, wobei der Körper (2) der Injektionsvorrichtung mindestens zwei Griffbereiche (12a, 12b) auf einer distalen Oberfläche (14) des Körpers der Vorrichtung umfasst, wobei mindestens einer der Griffbereiche (12a) mit einem Sensor (16a) versehen ist und die Auflage des Zeigefingers, des Mittelfingers, des Ringfingers oder des kleinen Fingers des Benutzers empfangen kann, und die Kolbenstange (3a) ein proximales Ende mit einem Griffbereich (12c) umfasst, wobei der Griffbereich (12c) mit einem Sensor (16c) versehen ist und die Auflage des Daumens des Benutzers empfangen kann, um ein Ergreifen der spritzenähnlichen Vorrichtung zu ermöglichen.

9. Vorrichtung (1) nach Anspruch 8, bei der der Griffbereich (12c) der Kolbenstange (3) an einem Abzug (15) positioniert ist, der fest mit der Kolbenstange verbunden ist und sich von einer Position distal zum proximalen Ende der Kolbenstange (3) schräg oder senkrecht zur Kolbenstange erstreckt.

10. Vorrichtung (1) nach einem der Ansprüche 1 bis 7, mit mindestens drei Griffbereichen (12a, 12b, 12c), die auf dem Körper (2) der Vorrichtung um eine Längsachse (B) der Vorrichtung herum angeordnet sind, wobei mindestens einer der Griffbereiche (12a) mit einem Sensor (16a) versehen ist, wobei die Griffbereiche (12a, 12b, 12c) jeweils die Auflage des Zeigefingers, des Mittelfingers oder des Daumens des Benutzers aufnehmen können, um ein Halten der stiftartigen Vorrichtung in der Hand zu ermöglichen.

11. Vorrichtung (1) nach einem der vorhergehenden Ansprüche, wobei das Halteelement des Behälters (40) eine schwenkbare Abdeckung (4) umfasst, die mit einer Öffnung (7) versehen ist, die mindestens das proximale Ende (43) des Behälters aufnehmen kann, wobei die schwenkbare Abdeckung (4) zwischen einer Injektionsposition, in der die Öffnung (7) mit dem Hub der Kolbenstange (3) ausgerichtet ist, und einer freien Position schwenkbar ist, in der die Öffnung (7) in Bezug auf den Hub der Kolbenstange (3) versetzt und für den Benutzer zugänglich ist, um das Einsetzen des Behälters (50) in die Öffnung zu ermöglichen.

12. Vorrichtung (1) nach einem der vorhergehenden Ansprüche, wobei das Halteelement des Behälters (40) zusätzlich eine Vielzahl von austauschbaren Ringen (5) mit Öffnungen (7) mit unterschiedlichen Durchmessern umfasst, die entsprechende Behälter (40) mit unterschiedlichen Durchmessern aufnehmen können.

13. Vorrichtung (1) nach Anspruch 12, bei der die schwenkbare Abdeckung (4) aus mehreren Teilen besteht, darunter mindestens ein erster Teil (10), der mit dem Körper (2) der Vorrichtung ein Ganzes bildet, und mindestens ein zweiter beweglicher Teil (11), der zwischen der Injektionsposition und der freien Position schwenken kann, wobei die beiden Teile (10, 11) der schwenkbaren Abdeckung (4) somit eine Zange mit variablem Abstand bilden, die es ermöglicht, den Behälter (40) aufzunehmen und zu umschließen, um ihn in einer festen Position zu halten.

14. Vorrichtung (1) nach Anspruch 13, bei der das zweite bewegliche Teil (11) vom Körper (2) der Vorrichtung abgenommen werden kann.

15. Vorrichtung (1) nach Anspruch 14, bei der der zweite bewegliche Teil (11) farbcodiert ist, um vom Benutzer identifiziert werden zu können, oder mit einer elektronischen Erkennungs- und Lesevorrichtung versehen ist, um dem Prozessor (18) die geometrischen oder rheologischen Eigenschaften des Behälters (40) und seines Inhalts anzuzeigen, damit die Erkennungsvorrichtung automatisch die abgegebene Dosis der Zusammensetzung für eine einheitliche Bewegung der Kolbenstange (3) berechnen kann.

16. Vorrichtung (1) nach einem der vorhergehenden Ansprüche, wobei das elektromechanische System einen Speicher umfasst, in dem Steuergesetze und/oder Schwellenwerte gespeichert sind.

17. Vorrichtung (1) nach einem der vorhergehenden Ansprüche, die ferner eine Benutzerschnittstelle (26) umfasst, die es dem Benutzer ermöglicht, die Betriebsparameter der Vorrichtung zu speichern und auszuwählen und eine Rückmeldung der Vorrichtung zu den Betriebsparametern bei der Verwendung der Vorrichtung zu erhalten.

## Claims

1. An assisted manual injection device (1) for injecting a composition contained in a container (40), wherein the container (40) is provided with a needle (46) at its distal end, and contains a piston (50), the injection device comprising:
- a body (2) comprising at least one gripping zone (12a) by which the body can be held in a user's hand,
- a holding element (4) of the container, configured to receive and hold the container (40) in a stationary manner relative to the body,
- an electromechanical system comprising:
• a piston rod (3) capable of being moved in translation in the container (40) relative to the body (2) of the device in order to move the piston (50),
• an actuation system (17) of the piston rod (3),
• a processor (18) capable of communicating with the actuation system (17),
the device (1) being **characterized in that** it comprises at least one force sensor (16a) positioned on the body at a gripping zone, the force sensor being configured to receive, as input, a mechanical effort F1 exerted on said sensor (16a) by the user during the injection of the composition, and to transmit to the processor (18) an output signal F2 that depends on the mechanical force,
and the processor (18) is configured to send an instruction to the actuation system (17) depending on the output signal from the sensor (16a) in order to actuate the piston rod (3) depending on a predetermined control law, in order to adjust the mechanical effort that needs to be applied by the user to the piston rod (3) in order to inject the composition.

2. The device (1) according to claim 1, wherein the actuation system (17) comprises an effort multiplication system configured to furnish to the piston rod (3) a mechanical effort F2 which is a multiple of the effort F1 exerted by the user on the sensor (16a), and wherein the control law is an effort multiplication law.

3. The device (1) according to claim 1 or claim 2, wherein the control law defines a movement of the piston rod (3) in a proximal direction along a predetermined distance D1 when the injection is interrupted and the mechanical effort F1 exerted by the user on the sensor (16a) is less than a threshold value F1ad determined during a specific duration Δt1.

4. The device (1) according to one of the preceding claims, wherein the piston rod (3) has a starting position, and the control law defines a movement of the piston rod in a proximal direction along a predetermined distance D2 when the injection is terminated and the mechanical effort F1 exerted by the user on the sensor (16a) is less than a threshold value F1ad determined during a specific duration Δt2 or when the piston rod is at the distal end of travel.

5. The device (1) according to one of the preceding claims, wherein the needle (46) being positioned in a subcutaneous zone of the human body, the control law defines a movement of the piston rod (3) in a proximal direction according to a predetermined distance D3 in order to perform a calibrated aspiration of a specific volume of a human fluid, in order to detect the possible presence of blood in the needle.

6. The device (1) according to one of the preceding claims, comprising a selective activation means (25) allowing the user to selectively activate or deactivate the control of the piston rod (3) by the processor (18) via the control law.

7. The device (1) according to claim 6, wherein the selective activation means (25) comprises an adjustment means allowing the user to choose a trigger threshold F1s, in such a way that the control of the piston rod (3) by the processor (18) via the control law is triggered only when the effort F1 exerted by the user on the sensor (16a) is greater than or equal to the trigger threshold F1s.

8. The device (1) according to one of the preceding claims, wherein the body (2) of the injection device comprises at least two gripping zones (12a, 12b) on a distal surface (14) of the body of the device, where at least one of the gripping zones (12a) is provided with a sensor (16a) and is able to receive pressure from the index finger, the middle finger, the ring finger or the little finger of the user, and the piston rod (3a) comprises a proximal end comprising a gripping zone (12c), said gripping zone (12c) being provided with a sensor (16c) and being able to receive the pressure from the user's thumb to allow the device to be held like a syringe.

9. The device (1) according to claim 8, wherein the gripping zone (12c) of the piston rod (3) is positioned on a trigger (15) integral with the piston rod and which extends obliquely or perpendicular to the piston rod from a distal position relative to the proximal end of said piston rod (3).

10. The device (1) according to one of claims 1 to 7, comprising at least three gripping zones (12a, 12b, 12c) arranged on the body (2) of the device around a longitudinal axis (B) of the device, at least one of said gripping zones (12a) being provided with a sensor (16a), said gripping zones (12a, 12b, 12c) being able to receive respectively the pressure from the user's index finger, middle finger, or thumb in order to allow the device to be held like a pen.

11. The device (1) according to one of the preceding claims, wherein the holding element of the container (40) comprises a pivotable cover (4) provided with an orifice (7) capable of receiving at least the proximal end (43) of the container, the pivotable cover (4) being capable of pivoting between an injection position in which the orifice (7) is aligned with the travel path of the piston rod (3), and a free position in which the orifice (7) is offset relative to the travel path of the piston rod (3) and is accessible by the user to allow the insertion of the container (50) into said orifice.

12. The device (1) according to one of the preceding claims, wherein the holding element of the container (40) further comprises a plurality of interchangeable rings (5) provided with orifices (7) of different diameters adapted to receive corresponding containers (40) of different diameters.

13. The device (1) according to claim 12, wherein the pivotable cover (4) is produced in several parts, of which at least a first part (10) is integral with the body (2) of the device, and at least a second movable part (11) capable of pivoting between the injection position and the free position, the two parts (10, 11) of the pivotable cover (4) thus forming a clamp of variable spacing making it possible to receive and clamp the container (40) in order to maintain it in a stationary position.

14. The device (1) according to claim 13, wherein the second movable part (11) is capable of being detached from the body (2) of the device.

15. The device (1) according to claim 14, wherein the second movable part (11) is color-coded to be identifiable by the user or is provided with a recognition and electronic reading device in order to specify to the processor (18) the geometric or rheological characteristics of the container (40) and its contents so that the recognition device can automatically calculate the dose of composition delivered for a unitary movement of the piston rod (3).

16. The device (1) according to one of the preceding claims, wherein the electromechanical system comprises a memory in which control laws and/or threshold values are recorded.

17. The device (1) according to one of the preceding claims, further comprising a user interface (26) allowing the user to record and select the operating parameters of the device, and to have information feedback from the device about said operating parameters during the use of the device.
